(19) Europäisches Patentamt
European Patent Office
Office européen des brevets

(11) EP 1 352 903 A1

(12) EUROPEAN PATENT APPLICATION
published in accordance with Art. 158(3) EPC

(43) Date of publication:
**15.10.2003 Bulletin 2003/42**

(21) Application number: **02729560.9**

(22) Date of filing: **11.01.2002**

(51) Int Cl.⁷: **C07D 233/92**, A61K 31/4164, A61K 9/08 // (A61P37/08, 27:00, 27:14, 27:16, 17:04)

(86) International application number:
**PCT/JP02/00151**

(87) International publication number:
**WO 02/055506 (18.07.2002 Gazette 2002/29)**

(84) Designated Contracting States:
**AT BE CH CY DE DK ES FI FR GB GR IE IT LI LU MC NL PT SE TR**

(30) Priority: **15.01.2001 JP 2001006700**

(71) Applicant: **SHOEI CO., LTD.**
**Fukuoka-shi, Fukuoka 812-0011 (JP)**

(72) Inventor: **NISHIMUTA, Kazuhiro**
**Fukuoka-shi, Fukuoka 812-0011 (JP)**

(74) Representative: **HOFFMANN - EITLE**
**Patent- und Rechtsanwälte**
**Arabellastrasse 4**
**81925 München (DE)**

# (54) PREVENTIVE OR REMEDIAL AGENT FOR ALLERGIC DISEASE AND MEDICAL WATER-BASED LIQUID PREPARATION, EYE DROP, AND NASAL DROP

(57) This invention provides an agent for the prevention or treatment of allergosis and a medical aqueous agent, an ophthalmic solution and a nasal drop for allergosis. The agents contain the nitroimidazole compound represented by the following formula (1) or a pharmaceutically acceptable salt thereof,

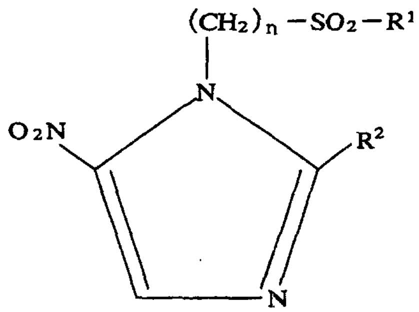

(1)

wherein $R^1$ and $R^2$ are selected independently from a straight or branched lower alkyl group having 1 to 6 carbon atoms which is optionally substituted, and "n" is an integer from 1 to 4.

EP 1 352 903 A1

## Description

FIELD OF THE INVENTION

The present invention relates to an agent for the prevention or treatment of allergosis, and a medical aqueous agent an ophthalmic solution, and a nasal drop for the prevention or treatment of allergosis, which agents comprise nitroimidazole compounds such as tinidazole or a pharmaceutically acceptable salt thereof. An agent for the prevention or treatment of allergosis according to the present invention is preferably used for allergosis of the eye or nose, and more preferably for allergic conjunctivitis or allergic rhinitis. In addition, a medical aqueous agent, an ophthalmic solution and a nasal drop for allergosis are used in the prevention or treatment of allergosis.

BACKGROUND OF THE INVENTION

Tinidazole(1-(2-ethylsulfonyl)ethyl-2-methyl-5-nitro-1H-imidazole) having a nitroimidazole skeleton is known to have anti-trichomonas activity, and is used for the treatment of infections caused by Trichomonas vaginalis as a medicine that can be administered either orally or inserted into the vagina. The compound is also effective against amebic dysentery and giardiasis, and can be administered as an internal remedy (oral formulation) or as a vaginal medicine.

For the treatment of allergosis such as allergic conjunctivitis or allergic rhinitis, aqueous agents,which contain steroids, non-steroidal anti-inflammatory drugs or anti-allergic drugs are also used.

DISCLOSURE OF THE INVENTION

The present invention provides an agent for the prevention or treatment of allergosis, an medical aqueous agent, ophthalmic solution and nasal drop for allergosis such as allergic conjunctivitis or allergic rhinitis, containing nitroimidazole compounds or a pharmaceutically acceptable salt thereof. The present inventor has conducted intensive studies to reach the above-mentioned objective, and has found that a nitroimidazole compound represented by tinidazole known as an antiprotozoal agent, or a pharmaceutically acceptable salt thereof, is effective as an agent for the prevention or treatment of allergosis such as allergic conjunctivitis or allergic rhinitis.

However, tinidazole is a crystal or crystal powder having low water solubility. Its solubility in a buffer solution (PH5 to 7) at 25°C is 0.5 to 0.4 weight/volume % (W/V%); and while tinidazole readily dissolves in organic solvents such as acetone, an organic solvent is not suitable for use as a carrier in a medical pharmaceutical preparation, especially in a an ophthalmic solution, nose drops, or the like, since such preparations are administrated locally to sensitive areas.

The present inventor has therefore conducted intensive studies to obtain a pharmaceutical preparation in the form of an aqueous solution in which tinidazole is dissolved at a sufficiently high concentration to exhibit efficacy, but which preparation remains low in acridity. As a result of these studies, the present inventor has found that tinidazole is able to be dissolved in the presence of a high concentration of a solubilizing agent in water, and that such an aqueous solution can be used as an ophthalmic solution or as nasal drop. Furthermore, a stable ophthalmic aqueous suspension or nasal drop can be provided in which neither aggregation nor caking of suspended particles occurs because tinidazole is a compound having low solubility in water. In addition, equivalent effects can be obtained by using a similar formulation of a nitroimidazole compound or a pharmaceutically acceptable salt thereof. Thus, the present invention has been completed.

The present invention is as follows.

[1] An agent for the prevention or treatment of allergosis containing nitroimidazole compound or a pharmaceutically acceptable salt thereof represented by the formula (1),

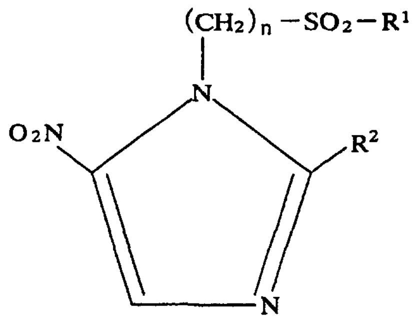

(1)

wherein $R^1$ and $R^2$ are selected respectively independently from a straight or branched lower alkyl group having 1 to 6 carbon atoms which is an optionally substituted group, and "n" is an integer from 1 to 4.

[2] An agent for the prevention or treatment of allergosis described in [1] wherein the allergosis includes diseases of the eye or nose.

[3] An agent for the prevention or treatment of allergosis described in [2], wherein the allergosis of the eye is allergic conjunctivitis and allergosis of the nose is allergic rhinitis.

[4] An agent for the prevention or treatment of allergosis of any one of [1] to [3], wherein the allergosis is accompanied by itching.

[5] An agent for the prevention or treatment of allergosis of any one of (1] to [4], wherein the agent for the prevention of or remedy is an aqueous agent.

[6] An agent for the prevention or treatment of allergosis of any one of [1] to [5], wherein the nitroimidazole compound is tinidazole( $R^1$=ethyl, $R^2$=methyl n=2).

[7] A medical aqueous agent containing a nitroimidazole compound or a pharmaceutically acceptable salt thereof represented by the formula (1),

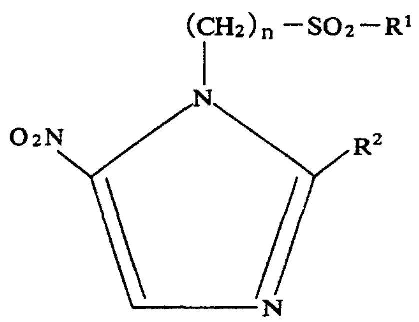

(1)

wherein $R^1$ and $R^2$ are selected respectively from a straight or branched lower alkyl group having 1 to 6 carbon atoms which is optionally substituted, and "n" is an integer from 1 to 4.

[8] A medical aqueous agent described in [7], wherein the agent contains a solubilizing agent.

[9] A medical aqueous agent of any one of [7] to [8], wherein the nitroimidazole compound is tinidazole ($R^1$=ethyl, $R^2$=methyl, n=2 ).

[10] An ophthalmic solution containing a nitroimidazole compound or a pharmaceutically acceptable salt thereof represented by the formula (1),

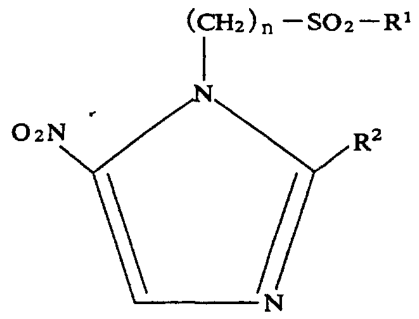

(1)

wherein $R^1$ and $R^2$ are selected respectively from a straight or branched lower alkyl group having 1 to 6 carbon atoms which is optionally substituted, and "n" is an integer from 1 to 4.

[11] An ophthalmic solution described in [10], wherein it contains solubilizing agent.

[12] An ophthalmic solution described in any one of [10] to [11], wherein the ophthalmic solution is an agent for the prevention of or remedy for allergosis.

[13] An ophthalmic solution described in any one of [10] to [12], wherein the nitroimidazole compound is tinidazole ( $R^1$=ethyl, $R^2$=methyl, n=2 ).

[14] Nasal drop containing a nitroimidazole compound or a pharmaceutically acceptable salt thereof represented by the formula (1),

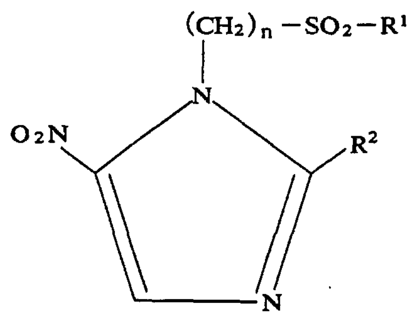

(1)

wherein $R^1$ and $R^2$ are selected respectively from a straight or branched lower alkyl group having 1 to 6 carbon atoms which is optionally substituted, and "n" is an integer from 1 to 4.

[15] Nasal drop described in [14], wherein the drops contain a solubilizing agent.

[16] Nasal drop described in any one of [14] to [15], wherein the nasal drop are an agent for the prevention of or remedy for allergosis.

[17] Nasal drop described in any one of [14] to [16], wherein the nitroimidazole compound is tinidazole ($R^1$=ethyl, $R^2$=methyl, n=2).

BEST MODE FOR CARRYING OUT THE PRESENT INVENTION

The active ingredient in the aqueous agent according to the present invention is a nitroimidazole compound or a pharmaceutically acceptable salt thereof ( hereafter referred to as "compound (1)") of the following formula :

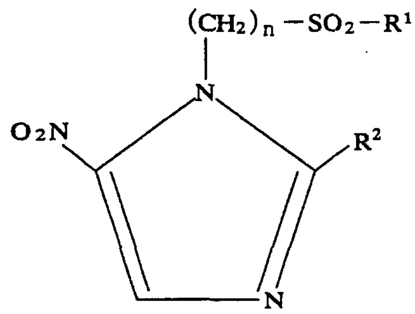

(1)

"The lower alkyl group of a straight chain or branched-chain having 1 to 6 carbon atoms which is optionally substituted as $R^1$ or $R^2$ may comprise, for example, methyl, ethyl, n-propyl, isopropyl, n-butyl, isobutyl, 2-butyl, 3-butyl, n-pentyl, isopentyl, neopentyl, n-hexyl, isohexyl and the like. Examples of substituent groups include halogen, nitro, cyano, hydroxy, which may be protected; an amino which may be protected; an alkylamino which may be protected; alkyl, alkoxy, carboxyl which may be protected; or alkoxycarbonyl, aryl, cycloalkyl, alkenyl and alkyl substituted by one or more halogen atoms. Examples of protective groups include any of those that can be used as a protective group for each of a hydroxy, amino, alkylamino and carboxyl group.

"N" is an integer from 1 to 4, preferably 2 or 3.

The term "pharmaceutically acceptable salt thereof" includes salts including inorganic acids such as hydrochloric acid, nitric acid, sulfuric acid and phosphoric acid; or organic acids such as acetic acid, citric acid, fumaric acid, maleic acid, terephthalic acid, lactic acid, butyric acid and benzoic acid.

The compound of formula (1) in which $R^1$= ethyl, $R^2$ = methyl and n=2, namely tinidazole (1-(2-ethylsulfonyl) ethyl-2-methyl-5-nitro-1H-imidazole) is the most preferable for use.

In the present invention, the term allergosis includes diseases considered to be caused by allergies as well as those that exhibit similar symptoms but which are unrelated to allergies, or are not demonstrated to be allergies. In addition, the term allergosis of the present invention includes allergosis for which antiallergic agents in the form of a drug for topical administration are applied.

As examples of allergosis, allergic conjunctivitis, spring catarrh, conjunctiva allergy which may be attributable to contact lens (giant papillary conjunctivitis), pollinosis, conjunctivitis phlyctaenulosa, contact blepharoconjunctivitis, Sjoegren syndrome, multiple corneal infiltrate, disciform deratitis, deep keratitis, cornea disorder flame, episcleritis, scleritis, uveitis,retinal vasculitis, optic disc vasculitis, optic neuritis, eosinophilic granuloma disease, rejection reaction caused by keratoplasty, pruritus of eye, allergic rhinitis, sneezing, nose itching, nose hypersensitivity, nasal vestibule eczema, rhinitis sicca anterior, nasal obstraction etc. can be mentioned.

An aqueous solution of the compound (1) of the present invention can be produced by dissolution of the Compound(1) in water in the presence of a solubilizing agent. Solubilizing agents include cationic surfactants, anionic surfactants, nonionic surfactants, etc.

Cationic surfactants include benzalkonium salts such as benzalkonium chloride(BAK),etc.

Anionic surfactants include sodium alkyl sulfates, etc., such as sodium dodecyl sulfate(SDS), pentane sodium sulphonate, octane sodium sulphonate, etc.

Nonionic surfactants of which HLB (hydrophile-lipophile balance) is 10 to 18 and a molecular weight is 500 to 4000 are preferable. Nonionic surfactants or ester type nonionic surfactants are preferable. For instance, polyoxyethylene sorbitan fatty acid esters such as polysorbate 80, polyoxyethylene hydrogenateded caster oils such as polyoxyethylene hydrogenateded caster oil 60(HCO 60), polyoxyethylene alkylphenyl formaldehyde condensate such as tyloxapol, polyoxyethylene polyoxypropylene block copolymers such as ploxamer and sucrose fatty acid esters are preferable.

The above-mentioned solubilizing agents may be used either singly or in combination. In the case that an aqueous solution containing an anionic surfactant is used as an ophthalmic solution, a nonionic surface active agent is preferably also used in order to decrease irritation, especially that which may occur in the eye due to the presence of anionic surfactants.

An appropriate concentration of compound (1) for use in a pharmaceutical aqueous preparation for medical treatment is determined. 0.001 w/v% or greater is preferable, and more preferably 0.01 w/v% or greater. More specifically, the concentration is 0.001~2 w/v%, preferably 0.01~2 w/v%, and more preferably 0.01~1 w/v%. When the concentration of the compound(1) is in the above-mentioned range, an aqueous solution of low acridity can be prepared, and efficacy of the drug can be demonstrated by topical application of an ophthalmic solution or a nasal drop.

In order to dissolve the compound(1) in an amount in the above-mentioned range in water, the aqueous solution preferably contains 0.5 to 10 W/V% of a solubilizing agent, and more preferably 0.5 to 4 W/V%.

Regarding the preferable ratio of the amount of the solubilizing agent to the compound(1): the cathionic surfactant is preferably 30 to 80 parts by weight to one part by weight of the compound (1), and more preferably 45 to 60 parts by weight; an anionic surfactant is preferably 3 to 10 parts by weight, and more preferably 4 to 7 parts by weight; and a nonionic surfactant is preferably 50 to 200 parts by weight, and more preferably 70 to 160 parts by weight.

On the other hand, when compound (1) is formulated as an aqueous suspension, suspending agents used in the present invention may include, for example, a nonionic surfactant, an anionic surfactant and a water-soluble high polymer, which may be used either singly or in combination.

Such nonionic surfactants are preferably those of which HLB is 10 to 18 and a molecular weight is 500 to 4000. Ether type nonionic surfactants and ester type nonionic surfactant are preferable. For instance, polyoxyethylene sorbitan fatty acid ester such as polysorbate 80, etc., polyoxyethylene hydrogenateded caster oils such as polyoxyethylene caster oil 60(HCO 60), polyoxyethylene alkylphenyl formaldehyde condensates such as tyloxapol, polyoxyethylene polyoxypropylene block copolymers such as ploxamer, etc., and sucrose fatty acid esters are preferable. In addition, anionic surfactants such as sodium lauryl sulfate(SLS), etc. can be used.

Water-soluble polymers that can be conveniently used include water-soluble cellulose compounds such as hydroxypropyl methylcellulose(HPMC), carboxymethylcellulose(CMC), methyl cellulose(MC), etc., carboxyvinylpolymer, macrogol, sodium chondroitin sulfate, polyvinylpyrrolidone such as polyvinylpyrrolidone K25(PVP K25), polyvinylpyrrolidone K30(PVP K30), polyvinylpyrrolidone K90(PVP K90), etc..

The concentration of the compound(1) in the suspension is usually 0.001 to 2 w/v%, preferably 0.01 to 2 w/v%, and more preferably 0.01 to 1 w/v%. When the concentration of the compound(1) is in the above mentioned range, a useful drug, which is efficacious when administered locally, and which is a stable suspension, can be prepared.

The suspending agent is preferably included from 0.00001 to 0.5 w/v% in the suspension. The surfactant and water-soluble polymer used as the suspending agent can be used either singly or in combination.

Regarding the concentration of each component of the suspending agent, in the case of a surfactant, the concentration is preferably 0.0001 to 0.1 w/v%, more preferably 0.001 to 0.1 w/v%, and most preferably 0.005 to 0.1 w/v%. In the case of a water-soluble polymer, the concentration is preferably 0.00001 to 0.5 w/v%, and still more preferably 0.0001 to 0.1 w/v%.

Regarding the ratio of the compound (1) to the surfactant, the surfactant is preferably present in an amount from 0.01 to 1 parts by weight to 1 part by weight of the compound (1).

Regarding the ratio of the compound (1) to the water-soluble polymer, the water-soluble polymer is preferably present in an amount from 0.0001 to 1 parts by weight to 1 part by weight of the compound (1), more preferably 0.0005 to 0.1 part by weight, and most preferably 0.001 to 0.01 part by weight to 1 part by weight of the compound (1). The ratio of from 0.001 to 0.01 parts by weight to 1 part by weight of the compound (1) is preferable, especially, when HPMC is used. A stable suspension with good dispersiveness can be prepared when a concentration of the suspending agent is in the above-mentioned range.

Other antiallergic agents such as histamine antireleaser, histamine receptor antagonist, leukotriene antireleaser, leukotriene receptor antagonist, PAF antireleaser, PAF receptor receptor antagonist, IgE antibody production inhibitor, cytokine antireleaser and cytokine receptor antagonist, etc. can be optionally mixed with the aqueous agent of the present invention.

When the medical aqueous agent of the present invention is used as an ophthalmic solution or a nasal drop, additional well-known additives which are usually used for an ophthalmic solution or nasal drop may be added. Such additives may include isotonic agent, buffering agent, chelating agent and preservative, etc.

Isotonic agents may include inorganic salts such as sodium chloride, boric acid, potassium chloride, etc.; and Polyhydric alcohols such as mannitol, sorbitol, etc..

Buffering agents may include a boric acid buffer solution, a phosphate buffer solution, an acetate buffer solution, a citrate buffer solution, a tris buffering agent, amino acids such as glutamine, ε-aminocaproic acid, etc.

Chelating reagents may include, for example, sodium Edetate, citric acid, etc.

Preservatives may include quaternary ammonium salts such as benzalkonium chloride, benzethonium chloride, etc; and paraminobenzoic acid esters such as paraminobenzoic acid metyl, ethyl paraminobenzoic acid, propyl paraminobenzoic acid, butyl paraminobenzoic acid, etc.; and sorbic acid, chlorobutanol, sodium edetate, boric acid, etc.

There are no particular restrictions as to the quantity of the additives; however, the concentration of an isotonic agent in an aqueous liquid is preferably 0.5 to 6.5 w/v%, that of a buffering agent is preferably 0.01 to 2 w/v%; 0.001 to 1 w/v% of a chelating reagent is preferable in an aqueous liquid; and 0.001 to 2 w/v% of a preservative is preferable in an aqueous liquid.

There are no particular restrictions on the preparative procedures for obtaining the medical aqueous agent, and it can be obtained by various well-known methods. For example, a buffering agent, isotonic agent, preservative, etc. are added to sterile, purified water, and dissolved. A solubilizing agent is added to the resulting solution, and then

the compound (1) is added and dissolved. At need, they may be heated up to about 70°C. After cooling, a pH modifier (hydrochloric acid, sodium hydroxide, etc.) are added, and an optional pH obtained; preferably in a range from 4.0 to 8.5, and more preferably in a range from 4.5 to 7.5.

Other preparative procedures for obtaining the medical aqueous agent can also be used. The compound (1) is dissolved in 0.5 to 10 w/v% cationic surfactant or anionic surfactant solution. A buffering agent, isotonic agent, preservative, etc. are added and dissolved, and then the resulting solution is freeze-dried. The resulting powder is dissolved in injection water etc. for use. Alternatively, compound (1), a buffering agent, isotonic agent, preservative, etc. are mixed using a mortar, etc. and then the mixture is enclosed in vials, etc. The resulting powder is dissolved for use in a solution containing the solubilizing agent. There are no particular restrictions on preparative procedures for the suspension, and any conventional well-known method can be used. For example, a suspending agent, buffering agent, isotonic agent and preservative may be added to sterile purified water and dissolved, optionally, with heating. To such a solution the compound(1) is added and uniformly suspended by use of any one of a homogenizer, mixer, mill or ultrasonication. The pH is then adjusted by using a pH modifier (hydrochloric acid, sodium hydroxide, etc.), and finally an aqueous suspension is obtained. A preferable pH range is in a range from 4.0 to 8.5, and more preferably is in a range from 4.5 to 7.5.

A medical aqueous agent according to the present invention as prepared above, nasal drop and ophthalmic solution are effective for the prevention or treatment of allergosis; especially, the aqueous agent can be locally administrated to the eye or nose of a mammal (human, rabbit, dog, cat, cattle, horse, monkey, etc.).

The dose of the drug of the present invention can be appropriately determined in accordance with an administration route, symptoms, age, body weight, etc, of a patient. For example, in a case that the drug is administered to an adult patient as an ophthalmic solution having allergosis, an amount of 1 to several drops each administration, 1 to 6 times a day would be desirable for an ophthalmic solution containing the compound(1) as an active ingredient in an amount of 0.001 to 2 w/v%.

Furthermore, an antiallergic agent, anti-inflammatory agent and/or an antimicrobe ophthalmic solution, nasal drop, etc. can be used jointly with the drug of the present invention.

## Examples

Following there is provided a more detailed explanation of the present invention using Examples and Test Examples, but the present invention is not limited thereto.

Effects of a tinidazole aqueous agent for allergosis of the eye or nose is shown in the following. The pharmaceutical preparation used for the Test was obtained as follows :

> Tinidazole, glycerin(solubilizing agent) and methyl parahydroxybenzoic acid(preservative) in the following amounts were weighed, and 70 ml of water was added and heated in a water bath up to 75°C and dissolved. Then, after cooling to room temperature, the total volume was adjusted to 100 ml by adding water.

Table 1

| Preparation and mashing quantity of tinidazole solution (unit : g/100 ml) | | | | |
|---|---|---|---|---|
| | Placebo | 0.01% solution | 0.1% solution | 1% solution |
| Tinidazole | - | 0.01 | 0.1 | 1 |
| Glycerin | 5 | 5 | 5 | 5 |
| Methyl Parahydroxy benzoic acid | 0.05 | 0.05 | 0.05 | 0.05 |
| injectable injectable water | an amount making up total 100 ml | an amount making up total 100 ml | an amount making up total 100 ml | an amount making up total 100 ml |

### Test Example 1: Effect of tinidazole on allergic conjunctivitis model

The anti-allergic action of tinidazole was evaluated using an experimental allergic conjunctivitis model(guinea pig).

(1) Test animal

In the test, Hartley male guinea pigs (each group consisting of 8 guinea pigs) at age 6 weeks were used.

(2) Preparation of guinea pig OA antiserum

Antiserum made by the following method was used: According to the method of Levine et al (J.Immunol., 106,29-33(1971)), the guinea pigs were sensitized with 4 times-administration of physiological saline 1 ml containing 10 μg of ovalbumin (hereafter called "OA") and 5 mg of aluminum hydroxide via the peritoneal cavity at intervals of 2 weeks. 1 week after the final sensitization, blood was sampled from the hind leg, and left at room temperature for 30 minutes. The blood was then left at 4°C for 4-5 hours, and serum was then collected by centrifugation at 1680 x 9.8 $m/s^2g$ for 15 minutes. The serum obtained was frozen and stored at -80°C.

(3) Measuring method

20 μl/site of Anti OA guinea pig serum (64 antibody titer in PCA (passive cutaneous anaphylaxis reaction)) was diluted with an equal amount of physiological saline. 20 μl/site of the solution was injected intradermally in binocular palpebral conjunctiva of the guinea pig under ether anesthesia. Thus, passive sensitization was carried out. After 7 days, an anaphylaxis reaction was induced by injecting 3 ml/kg of a mixture consisting of equal amounts of liquid 1% Evans blue physiological saline and 2% OA physiological saline into the vein. Thirty minutes after the reaction provocation, the guinea pig was sacrificed by bleeding, and binocular palpebral conjunctivas were then extracted and put in a test tube, after which 2 ml of 1 mol/ml potassium hydroxide was added, and the mixture left to stand at 37°C for 48 hours.

Then, 8 ml of 0.6 mol/ml of liquid mixture of phosphoric acid and acetone(5 to 13) was added and shaken; thereafter, the mixture was stirred using a homogenizer, and centrifuged at 1680 x $9.8/s^2$ for 15 minutes. The leakage pigment quantity was colorimetered at 620nm using a spectrophotometer.

As a subject material, placebo solution, 0.01% tinidazole solution, 0.1% tinidazole solution and 1% tinidazole solution were used. 50 μL of the subject material was placed into one eye, both 15 minutes and 5 minutes before administration of OA, which is an antigen.

The results are shown in Table 2.

Table 2

| Test group | No. | Quantity of dye leakage (μg/site) |
|---|---|---|
| Nonsensitization (Placebo) | 8 | 10.15 ± 0.68 |
| Sensitization Control (Placebo) | 8 | 67.38 ± 4.22** |
| 0.01% tinidazole solution | 8 | 31.48 ± 2.28## |
| 0.1% tinidazole solution | 8 | 24.99 ± 4.14## |
| 1% tinidazole solution | 8 | 15.02 ± 3.20## |

**: p < 0.01 (There is a significant difference between the nonsensitized group and sentitized group, t-test)

##: p < 0.01 (There is a significant difference in comparison with the sensitization control group, Dunnett's d-test)

As shown above, tinidazole showed a significant inhibitory effect at each concentration. Further, a dose relationship could be confirmed; namely a dye leakage quantity decreases with a rise in concentration of tinidazole. Under the test conditions employed, tinidazole solution exhibits an inhibitory effect on allergic conjunctivitis.

Test example 2: The anti-allergic action of tinidazole was evaluated using experimental allergic rhinitis model(guinea pigs).

(1) Test animal

In the test Hartley male guinea pigs(one group consisting of 8 guinea pigs) at age 8 weeks were used.

(2) Preparation of guinea pig OA antiserum

An antiserum made by the following method was used:

According to the method (J. Immunol., 116,392-397(1976)) of Orange and Moore et al., 20 mg of ovalbumin

(hereafter called "OA") was dissolved in 10 ml of physiological saline and then mixed with an equal amount of Freund's complete adjuvant. The guinea pigs were sensitized by using a subcutaneous injection of the 1 ml of the solution once every 4 days, a total of 4 times. After 3 weeks from the final sensitization day, blood was sampled from the abdominal aorta and left at room temperature for 30 minutes; furthermore, it was left at around 4°C for 4 to 5 hours. The serum was then collected using centrifugal separation at 1680 x 9.8 $m/s^2$ for 15 minutes. The collected serum was frozen and preserved at -20°C.

(3) Nasal resistance rise reaction measurement of passively sensitized guinea pigs

1 ml of 1% OA physiological saline (antigen liquid) was injected into the passively sensitized guinea pigs from the trigonal cock to the nasal cavity, and extra antigen liquid was discharged after being worked for 1 minute. Then, a nasal resistance rise reaction measurement was carried out( Konzett and Rossler method : Naunyn-Schmledebergs Arch. Exp. Pathol. Pharmakol., 195, 71 (1940)).

Namely, guinea pig anti OA serum( 8192 antibody titers) of 0.3 ml per guinea pig was administered in auricular veins of guinea pigs for sensitization. After 24 to 26 hours of sensitization, passively sensitized guinea pigs were anesthetized using urethane, after which tracheae was exposed by cervix median incision.

A polyethylene tube was inserted in the nasal cavity side from tracheostomy division and ligated. A polyethylene cannula was fixed for respiration maintenance in the trachea side, and a Y-shaped cannula, which was also connected the trigonal cock with the nasal cavity side in the inserted polyethylene cannula, was fixed.

A Harvard type respirator was connected to one end of a T-shaped cannula, and 5 ml air was provided at 60 beats/minute. Ventilation over flow quantity at a constant pressure load (10 cm, $H_2O$) from the other end of the Y-shaped cannula was induced to the Bronchospasm Transducer (7020, Ugobasile) as nasal resistance. Changes in ventilation overflow quantity were recorded on the recorder through a bioelectricity amplifier (AB-621G, Nihon Kohden Corporation) until after 20 minutes of nasal cavity injection of OA.

The following materials were used: Placebo solution, 0.01% tinidazole solution, 0.1% tinidazole solution and 1% tinidazole solution.

Instillation, using an assay atomizer for instillation of nose drop, was carried out total of 4 times for 2 times each at 10 minute and 5 minute before injection of OA, which is an antigen, to right and left nasal cavities.

(4) Summary and statistical method of the result

An undulate height (cm) on the recording paper was measured using calipers, and a nasal resistance value was obtained by the following equation, by which a mean value and standard error were calculated:

The results are shown in table 3.

(Equation 1)

nasal resistance value(%) = (measured value(cm)- value before administration(cm))÷(value at perfect closure(cm)- value before administration(cm)) x 100

Table 3

| Effect of the rise in the nasal resistance of passive sensitized cavities | | | | | | | | |
|---|---|---|---|---|---|---|---|---|
| Test group | No. | The rate-of-climb of the nasal resistance | | | | | | |
| | | | 1min. | 5min. | 10min | 15min. | 20min. |
| Non-sensitization (Placebo) | | Mean | 0.10 | -0.53 | 0.28 | 0.13 | -0.35 |
| | 8 | S.E. | 1.28 | 1.97 | 1.78 | 2.19 | 2.60 |

Table 3 (continued)

| Effect of the rise in the nasal resistance of passive sensitized cavities | | | | | | | |
|---|---|---|---|---|---|---|---|
| Test group | No. | The rate-of-climb of the nasal resistance | | | | | |
| | | | 1min. | 5min. | 10min | 15min. | 20min. |
| Sensitization control (placebo) | | | * | ** | ** | ** | ** |
| | 8 | Mean | 11.44 | 23.67 | 26.04 | 26.41 | 27.77 |
| | | S.E. | 3.79 | 1.90 | 2.53 | 2.24 | 2.59 |
| 0.01% Tinidazole solution | | | | ## | ## | ## | ## |
| | 8 | Mean | 3.00 | 5.86 | 8.24 | 7.26 | 9.55 |
| | | S.E. | 1.87 | 1.56 | 1.96 | 3.14 | 2.54 |
| 0.1% Tinidazole solution | | | | ## | ## | ## | ## |
| | 8 | Mean | 4.90 | 7.75 | 9.02 | 11.71 | 13.96 |
| | | S.E. | 2.20 | 2.09 | 2.21 | 2.21 | 2.24 |
| 1% Tinidazole solution | | | | ## | ## | ## | ## |
| | 8 | Mean | 2.45 | 7.71 | 9.36 | 9.96 | 10.05 |
| | | S.E. | 2.09 | 2.37 | 2.60 | 2.75 | 2.21 |

*:$p < 0.05$, **: $p < 0.01$ (There is a significant difference between the non-sensitization group and sensitization group, t-test)
#:$p < 0.05$, ##: $p < 0.01$ (There is a significant difference in comparison with the sensitization control group, Dunnett's d-test)

As can be seen in Table 3, at each concentration of tinidazole there was a significant inhibitory effect; therefore, it is apparent that tinidazole solution has an inhibitory effect on allergic rhinitis.

Example 1 (prescription example of ophthalmic solution)

Sodium dodecyl sulfate,polysorbate 80, sodium dihydrogen phosphate dihydrate. boric acid and benzalkonium were dissolved in sterile purified water. After pH was adjusted to about 7 using a pH modifier, tinidazole was added thereto and heated up to around 70°C and dissolved. After cooling, the pH was adjusted to around 7 by using the pH modifier, whereby an ophathalmic solution was obtained:

| | |
|---|---|
| tinidazole | 0.1 g |
| sodium dodecyl sulfate | 0.65 g |
| polysorbate 80 | 3.25 g |
| sodium dihydrogen phosphate dihydrate | 0.1 g |
| boric acid | 1.6 g |
| benzalkonium chloride | 0.005 g |
| sodium hydroxide | appropriate amount |
| sterile purified water | an amount making up the total of 100 ml |
| pH | 7.0 |

Example 2 (prescription example of nasal drop)

With the following components, a nasal drop was obtained by using the same procedure as described in Example 1 :

| | |
|---|---|
| tinidazole | 0.05 g |
| polysorbate 80 | 4.0 g |
| sodium dihydrogen phosphate • dihydrate | 0.1 g |
| sodium chloride | 0.9 g |

(continued)

| | |
|---|---|
| benzalkonium chloride | 0.01 g |
| sodium hydroxide | appropriate amount |
| sterile purified water | an amount providing the total of 100 ml |
| pH | 8.0 |

Example 3 ( prescription example of ophthalmic solution )

With the following components, an ophthalmic solution was obtained by using the same procedure as described in Example 1 :

| | |
|---|---|
| tinidazole | 0.05 g |
| octane sodium sulphonate | 0.5 g |
| polysorbate 80 | 3.35 g |
| sodium dihydrogen phosphate • dihydrate | 0.1 g |
| sodium chloride | 0.9 g |
| benzalkonium chloride | 0.005 g |
| sodium hydroxide | appropriate amount |
| sterile purified water | an amount making up the total of 100 ml |
| pH | 7.0 |

Example 4 ( prescription example of ophthalmic solution )

With the following components, an ophthalmic solution was obtained by using the same procedure as described in Example 1 :

| | |
|---|---|
| tinidazole | 0.05 g |
| pentane sodium sulphonate | 0.5 g |
| polysorbate 80 | 4.15 g |
| sodium dihydrogen phosphate dihydrate | 0.1 g |
| concentrated glycerin | 2.6 g |
| benzalkonium chloride | 0.005 g |
| sodium hydroxide | appropriate amount |
| sterile purified water | an amount making up the total of 100 ml |
| pH | 7.0 |

Example 5 (prescription example of ophthalmic solution)

With the following components, an ophthalmic solution was obtained by using the same procedure as described in Example 1 :

| | |
|---|---|
| tinidazole | 0.1 g |
| SDS | 0.65 g |
| polysorbate 80 | 3.25 g |
| sodium acetate | 0.1 g |
| sodium chloride | 0.9 g |
| methylparaben | 0.026 g |
| propylparaben | 0.014 g |
| hydrochloric acid | appropriate amount |
| sterile purified water | an amount making up the total of 100 ml |
| pH | 5.0 |

Example 6

With the following components, an ophthalmic solution or nasal drop was obtained by using the same procedure as described in Example 1 :

| | |
|---|---|
| tinidazole | 0.1 g |
| polysorbate 80 | 0.1 g |
| sodium phosphate • dihydrate | 0.1 g |
| sodium chloride | 0.9 g |
| benzalkonium chloride | 0.005 g |
| sodium hydroxide | appropriate amount |
| sterile purified water | an amount making up the total of 100 ml |
| pH | 7.0 |

Example 7

With the following components, an ophthalmic solution or nasal drop was obtained by using the same procedure as described in Example 1 :

| | |
|---|---|
| tinidazole | 1.0 g |
| HPMC | 0.001 g |
| sodium dihydrogen phosphate dihydrate | 0.1 g |
| sodium chloride | 0.9 g |
| benzalkonium chloride | 0.005 g |
| sodium hydroxide | appropriate amount |
| sterile purified water | an amount making the total 100 ml |
| pH | 7.0 |

Example 8

Using the following components, an ophthalmic solution or nasal drop was obtained by using the same procedure as described in Example 1 :

| | |
|---|---|
| tinidazole | 0.1 g |
| HCO-60 | 0.1 g |
| sodium dihydrogen phosphate • dihydrate | 0.1 g |
| sodium chloride | 0.9 g |
| benzalkonium chloride | 0.005 g |
| sodium hydroxide | appropriate amount |
| sterile purified water | an amount providing the total of 100 ml |
| pH | 7.0 |

Example 9

With the following components, an ophthalmic solution or nasal drop was obtained by using the same procedure as described in Example 1 :

| | |
|---|---|
| tinidazole | 1.0 g |
| HPMC | 0.01 g |
| sodium dihydrogen phosphate dihydrate | 0.1 g |
| sodium chloride | 0.9 g |
| methylparaben | 0.026 g |
| propylparaben | 0.014 g |

(continued)

| | |
|---|---|
| sodium hydroxide | appropriate amount |
| sterile purified water | an amount making the total 100 ml |
| pH | 5.0 |

Example 10

With the following components, an ophthalmic solution or nasal drop was obtained in the same procedure as described in Example 1 :

| | |
|---|---|
| tinidazole | 1.0 g |
| HPMC | 0.01 g |
| Boric acid | 1.6 g |
| benzalkonium chloride | 0.005 g |
| sodium hydroxide | appropriate amount |
| sterile purified water | an amount making the total 100 ml |
| pH | 7.0 |

Example 11

With the following components, an ophthalmic solution or nasal drop was obtained by using the same procedure as described in Example 1 :

| | |
|---|---|
| tinidazole | 0.1 g |
| tyloxapol | 0.1 g |
| sodium dihydrogen phosphate dihydrate | 0.1 g |
| sodium chloride | 0.9 g |
| benzalkonium chloride | 0.005 g |
| sodium hydroxide | appropriate amount |
| sterile purified water | an amount making the total 100 ml |
| pH | 7.0 |

Example 12

With the following components, an ophthalmic solution or nasal drop was obtained by using the same procedure as described in Example 1 :

| | |
|---|---|
| tinidazole | 0.1 g |
| SDS | 0.001 g |
| sodium dihydrogen phosphate • dihydrate | 0.1 g |
| boric acid | 1.6 g |
| benzalkonium chloride | 0.005 g |
| sodium hydroxide | appropriate amount |
| sterile purified water | an amount making the total 100 ml |
| pH | 7.0 |

Example 13

With the following components, an ophthalmic solution or nasal drop was obtained by using the same procedure as described in Example 1 :

| | |
|---|---|
| tinidazole | 1.0 g |
| methyl cellulose | 0.001 g |

(continued)

| | |
|---|---|
| sodium dihydrogen phosphate • dihydrate | 0.1 g |
| sodium chloride | 0.9 g |
| benzalkonium chloride | 0.005 g |
| sodium hydroxide | appropriate amount |
| sterile purified water | an amount making the total 100 ml |
| pH | 7.0 |

Example 14

With the following components, an ophthalmic solution or nasal drop was obtained by using the same procedure as described in Example 1 :

| | |
|---|---|
| tinidazole | 0.1 g |
| methyl cellulose | 0.1 g |
| sodium acetate | 0.1 g |
| concentrated glycerin | 2.6 g |
| metylparaben | 0.026 g |
| propylparaben | 0.014 g |
| chlorobutanol | 0.2 g |
| sodium hydroxide | appropriate amount |
| sterile purified water | an amount making the total amount 100 ml |
| pH | 5.0 |

Example 15

With the following components, an ophthalmic solution or nasal drop was obtained by using the same procedure as described in Example 1 :

| | |
|---|---|
| tinidazole | 0.1 g |
| sucrose fatty acid ester | 0.05 g |
| sodium dihydrogen phosphate dihydrate | 0.1 g |
| sodium chloride | 0.9 g |
| benzalkonium chloride | 0.005 g |
| sodium hydroxide | appropriate amount |
| sterile purified water | an amount making the total 100 ml |
| pH | 7.0 |

Example 16

With the following components, an ophthalmic solution or nasal drop was obtained by using the same procedure as described in Example 1 :

| | |
|---|---|
| tinidazole | 0.1 g |
| polysorbate 80 | 0.005 g |
| PVP K30 | 0.2 g |
| sodium dihydrogen phosphate • dihydrate | 0.1 g |
| sodium chloride | 0.9 g |
| benzalkonium chloride | 0.005 g |
| sodium hydroxide | appropriate amount |
| sterile purified water | an amount making the total 100 ml |
| pH | 7.0 |

Example 17

With the following components, an ophthalmic solution or nasal drop was obtained by using the same procedure as described in Example 1 :

| | |
|---|---|
| tinidazole | 1.0 g |
| HPMC(2910) | 0.004 g |
| sodium dihydrogen phosphate•dihydrate | 0.1 g |
| sodium chloride | 0.9 g |
| benzalkonium chloride | 0.005 g |
| sodium hydroxide | appropriate amount |
| sterile purified water | an amount making the total 100 ml |
| pH | 7.0 |

INDUSTRIAL APPLICABILITY

An agent of the present invention exhibits excellent prevention of or therapeutic effect on allergic diseases such as allergic conjunctivitis, spring catarrh, conjunctiva allergy which originates from contact lens (giant papillary conjunctivitis), conjunctivitis phlyctaenulosa, contact blepharoconjunctivitis, Sjoegren syndrome, multiple corneal infiltrate, discoidal keratitis, deep keratitis, cornea disorder flame, episcleritis, scleritis, uveitis, retinal vasculitis, optic disc vasculitis, optic neuritis, eosinophilic granuloma disease, rejection reaction caused by keratoplasty, pruritus of eye, allergic rhinitis, sneeze, nose itching, nose hypersensitivity, nasal vestibule eczema, rhinitis sicca anterior, nasal obstruction. A agent is used preferably for allergosis of eye or nose, and more preferably for allergic conjunctivitis or allergic rhinitis. In addition, an agent according to the present invention has an excellent inhibitory action on itching, and is useful as an itching-suppressant. In addition, a medical aqueous agent, ophthalmic solution and nasal drop according to the present invention are useful as an agent for the prevention or treatment of allergosis.

## Claims

1. An agent for the prevention or treatment of allergosis comprising nitroimidazole compound represented by the formula (1)

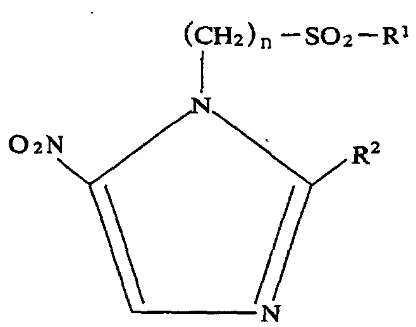

(1)

or a pharmaceutically acceptable salt thereof, 1
wherein $R^1$ and $R^2$ are selected independently from a straight or branched lower alkyl group having 1 to 6 carbon atoms which alkyl is optionally substituted, and "n" is an integer from 1 to 4.

2. An agent for the prevention or treatment of allergosis of Claim 1, wherein said allergosis is a disease of eye or nose.

3. An agent for the prevention or treatment of allergosis of Claim 2, wherein said allergosis of the eye is allergic conjunctivitis and said allergosis of the nose is allergic rhinitis.

4. An agent for the prevention or treatment of allergosis of any one of Claims 1 to 3, wherein said allergosis is accompanied by itching.

5. An agent for the prevention or treatment of allergosis of any one of Claims 1 to 4, wherein said agent is an aqueous agent.

6. An agent for the prevention or treatment of allergosis of any one of Claims 1 to 5, wherein the nitroimidazole compound is tinidazole($R^1$=ethyl, $R^2$=methyl n=2).

7. A medical aqueous agent containing a nitroimidazole compound represented by the formula(1)

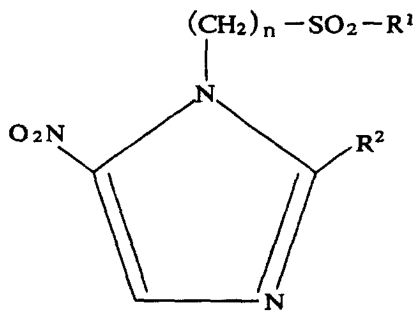

(1)

or a pharmaceutically acceptable salt thereof, 1
wherein $R^1$ and $R^2$ are selected independently from a straight or branched lower alkyl group having 1 to 6 carbon atoms which is optionally substituted, and "n" is an integer from 1 to 4.

8. A medical aqueous agent of Claim 7, wherein said aqueous agent contains a solubilizing agent.

9. A medical aqueous agent of any one of Claims 7 to 8, wherein the nitroimidazole compound is tinidazole ($R^1$=ethyl, $R^2$=methyl n=2).

10. An ophthalmic solution containing the nitroimidazole compound represented by the formula (1)

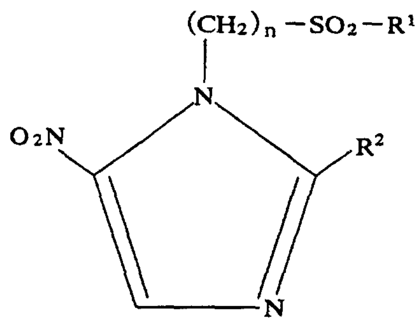

(1)

or a pharmaceutically acceptable salt thereof,
wherein $R^1$ and $R^2$ are selected independently from a straight or branched lower alkyl group having 1 to 6 carbon atoms which is optionally substituted, and "n" is an integer from 1 to 4.

11. An ophthalmic solution of Claim 10, wherein said solution contains a solubilizing agent.

12. An ophthalmic solution of any one of Claims 10 to 11, wherein the solution is an agent for the prevention or treatment of allergosis.

**13.** An ophthalmic solution of any one of Claims 10 to 12, wherein the nitoroimidazole compound is tinidazole($R^1$=ethyl, $R^2$=methyl, n=2).

**14.** Nasal drop containing the nitroimidazole compound represented by the Formula (1)

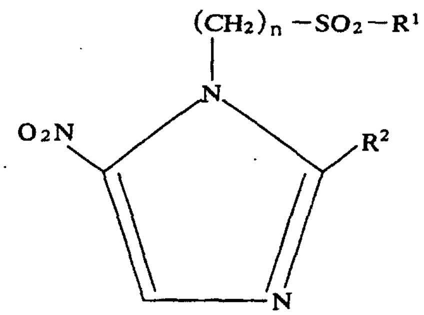

(1)

or a pharmaceutically acceptable salt thereof,
wherein $R^1$ and $R^2$ are selected independently from a straight or branched lower alkyl group having 1 to 6 carbon atoms which is optionally substituted, and "n" is an integer from 1 to 4.

**15.** Nasal drop of Claim 14, wherein said drop contains a solubilizing agent.

**16.** Nasal drop of any one of Claims 14 to 15, wherein the drop is an agent for the prevention or treatment of allergosis.

**17.** Nasal drop of any one of Claims 14 to 16, wherein the nitoroimidazole compound is tinidazole($R^1$=ethyl, $R^2$=methyl n=2).

**INTERNATIONAL SEARCH REPORT**

International application No.
PCT/JP02/00151

A. CLASSIFICATION OF SUBJECT MATTER
Int.Cl$^7$ C07D233/92, A61K31/4164, 9/08//A61P37/08, 27/00, 27/14, 27/16, 17/04

According to International Patent Classification (IPC) or to both national classification and IPC

B. FIELDS SEARCHED

Minimum documentation searched (classification system followed by classification symbols)
Int.Cl$^7$ C07D233/92, A61K31/4164, 9/08

Documentation searched other than minimum documentation to the extent that such documents are included in the fields searched

Electronic data base consulted during the international search (name of data base and, where practicable, search terms used)
CA(STN)

C. DOCUMENTS CONSIDERED TO BE RELEVANT

| Category* | Citation of document, with indication, where appropriate, of the relevant passages | Relevant to claim No. |
|---|---|---|
| A | US, 4675337, A (Merck & Co., Inc.), 23 June, 1987 (23.06.87), (Family: none) | 1-17 |
| A | US, 3689592, A (Pfizer Inc.), 05 September, 1972 (05.09.72), & JP 49-41345 B | 1-17 |
| A | US, 5256684, A (The Procter & Gamble Co.), 26 October, 1993 (26.10.93), & JP 62-48623 A | 1-17 |

☐ Further documents are listed in the continuation of Box C.  ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 March, 2002 (18.03.02) | 26 March, 2002 (26.03.02) |

| Name and mailing address of the ISA/ | Authorized officer |
|---|---|
| Japanese Patent Office | |
| Facsimile No. | Telephone No. |

Form PCT/ISA/210 (second sheet) (July 1998)